# DEMANDE DE BREVET EUROPEEN

(11) **EP 1 535 638 A1**
(43) Date de publication de la demande: **01.06.2005**
(21) Numéro de dépôt: 04292670.9
(22) Date de dépôt: 10.11.2004
(51) Int. Cl.: A61M 5/14, A61M 5/40

(54) **Dipositif de perfusion comprenant une poche munie d'un moyen d'obturation reversible**

(30) Priorité: 28.11.2003 FR 0350941
(71) Demandeur: MACO PHARMA, 59420 Mouvaux (FR)
(72) Inventeur: Brinon, Thierry, 95300 Pontoise (FR)
(74) Mandataire: Geismar, Thierry

(57) **Abrégé**

L'invention propose un dispositif de perfusion (1) formant un système unitaire en circuit fermé, comprenant une première poche (2) contenant ou destinée à contenir une première solution, une deuxième poche (3) contenant ou destinée à contenir une deuxième solution, et des moyens de perfusion (4), ledit dispositif comprenant en combinaison :
- des moyens de sélection à commande externe (10) aptes à sélectionner le passage, dans les moyens de perfusion (4), de la solution contenue dans la première poche (2) ou le passage de la deuxième solution dans la première poche (2) ;
- un moyen d'obturation réversible (11) de l'orifice d'entrée/sortie (6) de la première poche (2), ledit moyen comprenant un organe flottant (12) qui est monté mobile, à l'intérieur de la première poche (2), en regard de l'orifice d'entrée/sortie (6) entre une position d'obturation dans l'ouverture dudit orifice et une position d'écoulement à distance dudit orifice.

## Description

L'invention concerne un dispositif de perfusion par gravité formant un système unitaire en circuit fermé, ainsi qu'un procédé de perfusion à l'aide d'un tel dispositif.

Elle s'applique typiquement à la perfusion par gravité destinée à permettre l'administration parentérale de solutions injectables à un patient. Dans un exemple de réalisation particulier, la solution à injecter contient un principe actif nécessitant un dosage précis, tel qu'un agent antimitotique ou antibiotique.

On connaît du document FR-2 794 983 un dispositif de ce type comprenant une poche de médicament, une poche de rinçage, des moyens de perfusion en communication fluidique avec lesdites poches et des moyens de sélection aptes à assurer le passage sélectif dans les moyens de perfusion de la solution médicamenteuse ou de rinçage. Dans un tel dispositif, la solution médicamenteuse est d'abord injectée au patient, puis on manoeuvre les moyens de sélection pour permettre le rinçage des moyens de perfusion à l'aide de la solution de rinçage.

Un problème qui se pose avec un tel dispositif est que, si le personnel soignant n'arrête pas la perfusion de la solution médicamenteuse avant que la poche de médicament ne soit complètement vide, l'air contenu dans celle-ci peut être introduit dans les moyens de perfusion. Dans ce cas, lors du rinçage, le volume d'air est administré au patient, provoquant ainsi une embolie gazeuse pouvant entraîner la mort du patient.

L'invention propose de perfectionner un tel dispositif de perfusion de sorte à empêcher toute administration d'air par l'intermédiaire des moyens de perfusion.

A cet effet, l'invention propose d'utiliser un moyen d'obturation réversible d'un orifice d'écoulement, ledit moyen comprenant un organe flottant.

Le document WO-00/67823 décrit un tel moyen qui est monté dans une chambre compte-gouttes. Ainsi, lorsque la solution médicamenteuse est complètement injectée et que la chambre compte-gouttes est vide, l'organe flottant vient obturer l'orifice de sortie de ladite chambre, évitant ainsi le passage d'air en aval vers le perfuseur.

L'utilisation d'une telle chambre compte-gouttes dans un dispositif de perfusion selon l'invention ne serait pas satisfaisante. En effet, lorsque l'organe flottant bloque l'orifice de sortie de la chambre compte-gouttes, la dépression générée par le fluide en aval de la chambre compte-gouttes, tout en assurant l'étanchéité, empêche l'organe de se remettre à flotter lors du réamorçage de la chambre compte-gouttes avec la solution de rinçage. Pour assurer le rinçage, il serait alors nécessaire de procéder à une manipulation externe pour débloquer l'organe flottant.

L'invention vise également à utiliser un organe d'obturation réversible dans un dispositif de perfusion, qui est agencé pour éviter toute manipulation externe du moyen d'obturation en vue de son rinçage.

A cet effet, et selon un premier aspect, l'invention propose un dispositif de perfusion formant un système unitaire en circuit fermé, comprenant une première poche contenant ou destinée à contenir une première solution, une deuxième poche contenant ou destinée à contenir une deuxième solution, et des moyens de perfusion en communication fluidique avec la première poche par l'intermédiaire d'une première tubulure branchée respectivement sur un orifice d'entrée/sortie de ladite première poche et sur un moyen de connexion sur lequel sont branchés les moyens de perfusion, la deuxième poche étant en communication fluidique avec la première poche par l'intermédiaire d'une deuxième tubulure branchée respectivement sur un orifice de sortie de ladite deuxième poche et sur le moyen de connexion, ledit dispositif comprenant en combinaison :
- des moyens de sélection à commande externe aptes à sélectionner le passage, dans les moyens de perfusion, de la solution contenue dans la première poche ou le passage de la deuxième solution dans la première poche;
- un moyen d'obturation réversible de l'orifice d'entrée/sortie de la première poche, ledit moyen comprenant un organe flottant qui est monté mobile, à l'intérieur de la première poche, en regard de l'orifice d'entrée/sortie entre une position d'obturation dans l'ouverture dudit orifice et une position d'écoulement à distance dudit orifice.

Selon un deuxième aspect, l'invention propose un procédé de perfusion à l'aide d'un dispositif selon le premier aspect, comprenant les étapes successives suivantes :
- l'organe flottant étant en position d'écoulement, commande des moyens de sélection pour perfuser la première solution depuis la première poche;
- à la fin de ladite perfusion, l'organe flottant étant en position d'obturation, commande des moyens de sélection pour faire passer de la deuxième solution depuis la deuxième poche vers la première poche en déplaçant l'organe flottant vers sa position d'écoulement ;
- commande des moyens de sélection pour perfuser la deuxième solution qui est contenue dans la première poche.

L'invention sera comprise grâce à la description qui suit en référence aux dessins annexés, illustrant divers modes de réalisation.

Les figures 1 à 3 représentent de façon schématique trois modes de réalisation d'un dispositif de perfusion selon l'invention.

Les figures 4 et 5 représentent partiellement et de façon schématique la première poche des figures 1 à 3, montrant l'organe flottant du moyen d'obturation réversible respectivement en position d'écoulement et en position d'obturation de l'orifice.

L'invention concerne un dispositif de perfusion par gravité, notamment destiné à permettre l'administration parentérale de solutions injectables à un patient.

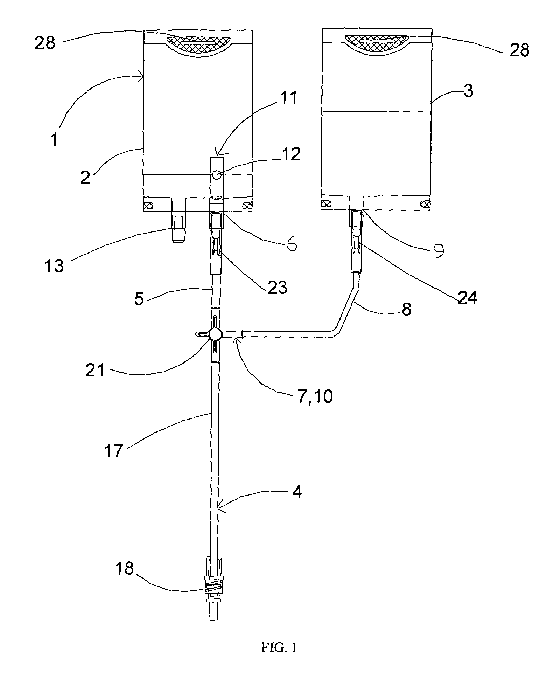

En référence aux figures 1 à 3, le dispositif de perfusion 1 forme un système unitaire en circuit fermé, comprenant une première poche 2 contenant ou destinée à contenir une première solution, une deuxième poche 3 contenant ou destinée à contenir une deuxième solution, et des moyens de perfusion 4.

Les moyens de perfusion 4 sont en communication fluidique avec la première poche 2 par l'intermédiaire d'une première tubulure 5 branchée respectivement sur un orifice d'entrée/sortie 6 de ladite première poche et sur un moyen de connexion 7 sur lequel sont branchés les moyens de perfusion 4.

La deuxième poche 3 est en communication fluidique avec la première poche par l'intermédiaire d'une deuxième tubulure 8 branchée respectivement sur un orifice de sortie 9 de ladite deuxième poche et sur le moyen de connexion 7.

Le dispositif 1 comprend en outre et en combinaison :
- des moyens de sélection 10 à commande externe aptes à sélectionner le passage, dans les moyens de perfusion 4, de la solution contenue dans la première poche 2 ou le passage de la deuxième solution dans la première poche 2 ; et
- un moyen d'obturation réversible 11 de l'orifice d'entrée/sortie 6 de la première poche 2, ledit moyen comprenant un organe flottant 12 qui est monté mobile, à l'intérieur de la première poche 2, en regard de l'orifice d'entrée/sortie 6 entre une position d'obturation dans l'ouverture dudit orifice et une position d'écoulement à distance dudit orifice 6.

Le dispositif de perfusion 1 selon l'invention constitue un système unitaire en circuit fermé, c'est-à-dire un système prêt-à-l'emploi, dont les différents éléments le constituant sont pré-connectés. Les éléments étant connectés de fabrication, on évite ainsi une étape de connexion préalablement à l'utilisation du système, qui constitue une manipulation supplémentaire entraînant une perte de temps et un risque de contamination.

Les solutions contenues dans les poches 2, 3 sont des solutions injectables. Elles peuvent être placées dans les poches au moment de la fabrication du dispositif ou introduites dans le dispositif de perfusion au moment de l'emploi par un orifice d'entrée/sortie de chacune des poches (non représenté).

Par exemple, la première poche contient une solution contenant un médicament, tel qu'un antibiotique ou un antimitotique et la deuxième poche contient une solution de rinçage. Des exemples de solution de rinçage comprennent des solutions de NaCl, glucose, eau ppi et solution de Ringer.

Selon un autre exemple, les deux poches 2, 3 sont pré-remplies d'une solution injectable de type solution de rinçage. En particulier, les deux poches contiennent la même solution. Au moment de l'emploi, un médicament est reconstitué dans la solution contenue dans la première poche 2 à l'aide d'un dispositif de transfert par l'intermédiaire d'un site d'injection 13 placé sur la première poche 2. Un dispositif de transfert approprié est décrit dans le document FR-A-2 790 749.

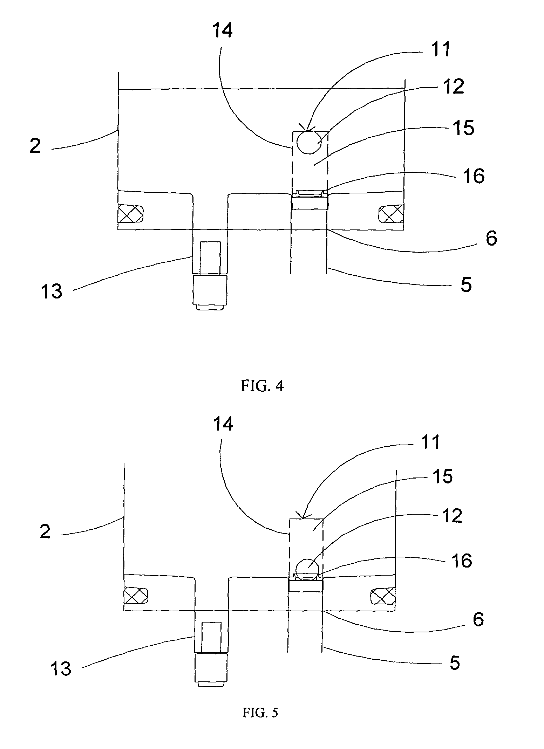

Un moyen d'obturation réversible 11 comprenant un organe flottant 12 est disposé dans la première poche 2. Lorsque la première poche 2 contient une solution, l'organe flottant 12 est à distance de l'ouverture de l'orifice d'entrée/sortie 6 de la première poche 2 (figure 4). Lorsque la première poche 2 est vide, l'organe flottant 12 obture ladite ouverture et empêche le passage d'air (figure 5) de la première poche 2 vers les moyens de perfusion 4.

L'organe flottant 12 possède un poids suffisant pour obturer de façon étanche l'ouverture de l'orifice d'entrée/sortie 6 de la première poche 2 et une densité lui permettant de flotter dans la solution contenue dans la première poche 2.

Notamment, l'organe flottant 12 est réalisé en un matériau plastique, par exemple en polypropylène.

Selon un mode de réalisation particulier, l'organe flottant 12 est formé d'une bille dont le diamètre est de l'ordre, tout en étant supérieur, à celui de l'ouverture de l'orifice d'entrée/sortie 6.

En référence aux figures 4 et 5, le moyen d'obturation 11 comprend un guide vertical ajouré 14 qui est prévu dans le prolongement de l'orifice d'entrée/sortie 6 de la première poche 2, dans lequel l'organe flottant 12 est logé mobile.

Le guide vertical 14 est ajouré de façon à permettre l'écoulement libre de la solution contenue dans la première poche 2 au travers de celui-ci.

Le guide vertical 14 est conçu de sorte à définir un compartiment intérieur 15 dans lequel l'organe flottant 12 est maintenu. L'organe flottant 12 se déplace librement dans ce compartiment 15. Le guide vertical 14 amène l'organe flottant 12 juste au dessus de l'ouverture de l'orifice 6 de la première poche 2 lorsque celle-ci se vide.

Selon une réalisation particulière, le guide vertical 14 est réalisé en un matériau thermoplastique tel que le polychlorure de vinyle ou le polyéthylène. Il est fabriqué sous forme de grille, tissé, non tissé ou feuille ajourée.

Selon une autre réalisation particulière, le guide vertical 14 est constitué par la première tubulure 5 s'étendant à l'intérieur de la première poche 2. L'extrémité de la tubulure disposée dans la poche 2 est fermée, par exemple par soudage. La partie s'étendant à l'intérieur de la poche 2 est ajourée.

Comme représenté sur les figures 4 et 5, le moyen d'obturation 11 comprend en outre un siège 16 disposé au niveau de l'ouverture de l'orifice d'entrée/sortie 6 de la première poche, sur lequel l'organe flottant se pose. Ce siège 16 empêche le passage de l'organe flottant 12 dans la première tubulure 5 et assure l'étanchéité de l'obturation de ladite ouverture.

Par exemple, le siège 16 comprend un joint d'étanchéité annulaire réalisé en un matériau polymère souple tel q'un élastomère de silicone. Le siège possède une zone d'étanchéité externe au niveau de l'orifice 6 de la première poche 2, et une zone d'étanchéité interne avec l'organe flottant 12 dans sa position d'obturation. La forme de la zone d'étanchéité interne est adaptée à la forme de l'organe flottant 12.

La présence de ce joint d'étanchéité associée à la dépression générée par l'écoulement de la solution en aval de la première poche assure l'étanchéité de l'obturation.

L'obturation de l'ouverture de l'orifice 6 de la première poche 2 est facilement réversible sans manipulation externe. Lorsque la première poche 2 est vide et en actionnant les moyens de sélection 10 de sorte à permettre le passage de la deuxième solution contenue dans la deuxième poche 3 vers la première poche 2, de la deuxième solution pénètre dans la première poche 2 par son orifice d'entrée/sortie 6 et soulève l'organe flottant 12 qui obturait cet orifice. L'organe flottant 12 est poussé par le dessous par la deuxième solution, libérant facilement l'orifice 6 de la première poche 2.

Lorsque, ensuite, la deuxième solution qui se trouve dans la première poche 2 est perfusée, l'organe flottant 12 se repositionne sur l'ouverture de l'orifice d'entrée/sortie 6 de la première poche jusqu'à sa position d'obturation.

En relation avec les figures 1 à 3, les moyens de perfusion 4 comprennent une troisième tubulure 17 dont une extrémité est connectée au moyen de connexion 7 et dont l'autre extrémité comprend un moyen de raccordement 18 au circuit veineux du patient, telle qu'une aguille ou un raccord pour cathéter, notamment un raccord mâle de type cône Luer.

Selon un mode de réalisation particulier, les moyens de perfusion 4 comprennent en outre une chambre compte-gouttes 19 qui est disposée sur la troisième tubulure 17.

La chambre compte-gouttes constitue un moyen de contrôle du débit de perfusion.

Notamment, la chambre de perfusion est munie d'un filtre à résidus 20 ayant une taille de pore de 15 µm. Le filtre 20 est disposé dans le fond de la chambre compte-gouttes 19.

Les moyens de perfusion 4 sont connectés à la première et deuxième tubulure 5, 8 par un moyen de connexion 7.

Des moyens de sélection 10 à commande externes sont aptes à sélectionner, dans les moyens de perfusion 4, de la solution contenue dans la première poche 2 ou le passage de la deuxième solution dans la première poche 2.

Selon un mode de réalisation, les moyens de sélection 10 sont en outre aptes à sélectionner le passage, dans les moyens de perfusion 4, de la deuxième solution lorsqu'elle est contenue dans la deuxième poche 3.

Selon un premier mode de réalisation représenté sur la figure 1, le moyen de connexion et les moyens de sélection sont intégrés sous la forme d'un robinet à voies multiples 21.

Le robinet 21 est notamment un robinet à trois voies raccordé à la première tubulure 5, à la deuxième tubulure 8 et aux moyens de perfusion 4.

Notamment, le robinet à trois voies possède trois possibilités d'écoulement. La première possibilité d'écoulement met en communication fluidique les moyens de perfusion 4 et la première poche 2 par l'intermédiaire de la première tubulure 5. Le chemin d'écoulement ainsi défini permet la perfusion de la solution contenue dans la première poches 2 au travers des moyens de perfusion 4.

La deuxième possibilité d'écoulement met en communication fluidique la première poche 2 et la deuxième poche 3 par l'intermédiaire, respectivement, de la première tubulure 5 et de la deuxième tubulure 8. Le chemin d'écoulement ainsi défini passe par l'orifice d'entrée/sortie 6 de la première poche 2 au regard duquel se trouve l'organe flottant 12.

La troisième possibilité d'écoulement met en communication fluidique les moyens de perfusion 4 et la deuxième poche 3 par l'intermédiaire de la deuxième tubulure 8. Le chemin d'écoulement ainsi défini permet le passage de la solution contenue dans la deuxième poche 3 au travers des moyens de perfusion 4.

Dans le cas où la première poche contient une solution médicamenteuse et la deuxième poche contient une solution de rinçage, ce chemin d'écoulement autorise la purge du dispositif de perfusion et de l'abord veineux du patient avec de ladite solution de rinçage avant administration de la solution médicamenteuse contenue dans la première poche 3.

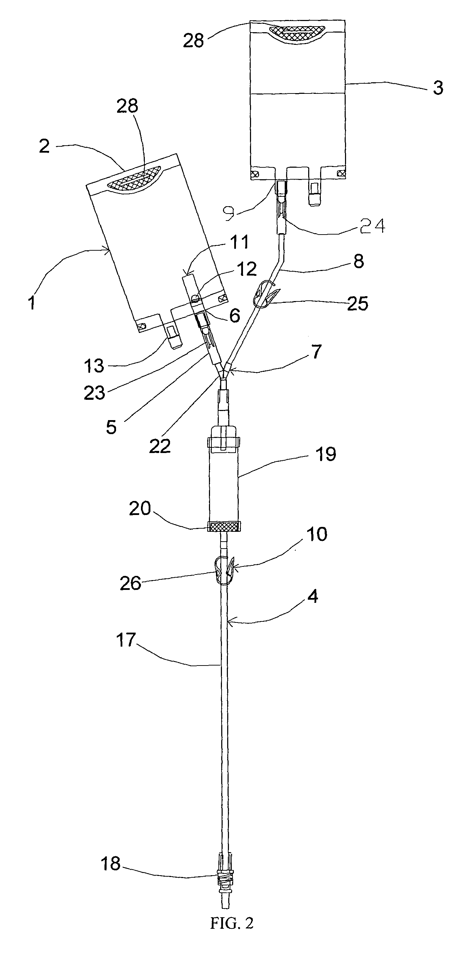

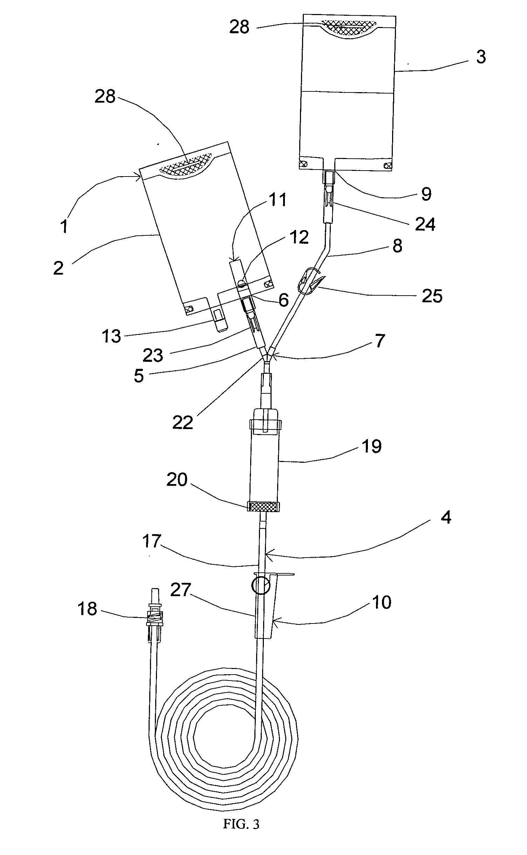

Selon les deux autres modes de réalisation représentés sur les figures 2 et 3, le moyen de connexion 7 est formé d'une jonction trois voies 22. Notamment, la jonction est un connecteur en Y ou en T.

Dans cette réalisation, les moyens de sélection 10 comprennent au moins un organe de pincement de type clamp ou roulette disposé sur une tubulure et/ou un organe sécable de type ouvre-circuit disposé dans une tubulure.

Notamment, les moyens de sélection comprennent deux ouvre-circuits 23, 24 disposés sur la première tubulure 5 et deuxième tubulure 8 au niveau des orifices 6, 9 des première et deuxième poche 2, 3, respectivement.

En combinaison avec ces ouvre-circuits 23, 24, les deuxième et troisième tubulures 8, 17 sont munies chacune d'un clamp 25, 26, respectivement.

Ainsi, pour assurer le passage dans les moyens de perfusion 4 de la solution contenue dans la première poche, l'ouvre-circuit 23 positionné sur la première tubulure 5 et le clamp 26 positionné sur la troisième tubulure 17 sont ouverts, et le clamp 25 positionnés sur la deuxième tubulure 17 est fermé.

Pour permettre le passage de la seconde solution vers la première poche 2, le clamp 25 situé sur la deuxième tubulure 8 et les deux ouvre-circuits 23, 24 sont ouverts et le clamp 26 situé sur la troisième tubulure 17 est fermé.

Dans ce cas, l'ouvre-circuit 23 est fermé et les deux clamps 25, 26 placés respectivement sur la deuxième tubulure 8 et la troisième tubulure 17 sont ouverts.

Dans le mode de réalisation représenté sur la figure 3, le clamp situé sur la troisième tubulure 17 est remplacé par un régulateur de débit à roulette 27 pouvant contrôler et stopper le débit de la solution à perfuser.

Dans le dispositif de perfusion 1, les poches et les tubulures du système sont réalisées notamment en un matériau thermoplastique souple et stérilisable tel que le polychlorure de vinyle ou une polyoléfine.

Notamment, les poches sont munies d'un orifice de suspension 28 de sorte à pouvoir suspendre lesdites poches sur un portique pour réaliser la perfusion par gravité.

Selon un deuxième aspect, l'invention propose un procédé de perfusion à l'aide d'un dispositif de perfusion 1, comprenant les étapes successives suivantes :
- l'organe flottant 12 étant en position d'écoulement, commande des moyens de sélection 10 pour perfuser la première solution depuis la première poche 2 ;
- à la fin de ladite perfusion, l'organe flottant 12 étant en position d'obturation, commande des moyens de sélection 10 pour faire passer de la deuxième solution depuis la deuxième poche 3 vers la première poche 2 en déplaçant l'organe flottant 12 vers sa position d'écoulement ;
- commande des moyens de sélection 10 pour perfuser la deuxième solution qui est contenue dans la première poche 2.

Dans un mode de réalisation particulier, le dispositif de perfusion 1 est disposé de telle sorte que les deux poches soient à la même hauteur. Par exemple, les deux poches sont suspendues à un portique au moyen des orifices de suspension 28.

Lors de la commande des moyens de sélection 10 pour faire passer une partie de la deuxième solution vers la première poche 2, la deuxième solution s'écoule automatiquement dans la première poche 2 jusqu'à ce que les niveaux des solutions dans les deux poches 2, 3 s'alignent. L'organe flottant 12 du moyen d'obturation est automatiquement déplacé dans sa position d'écoulement, grâce à la contre-pression exercée dans l'orifice d'entrée/sortie 6 de la première poche en aval dudit organe flottant 12, lors de la phase de transfert de la solution contenue dans la deuxième poche 3 vers la première poche 2.

Le procédé selon l'invention permet de perfuser deux solutions injectables successivement sans risque d'administrer de l'air. En effet, l'air susceptible de passer dans les moyens de perfusion 4, c'est-à-dire l'air contenu dans la première poche 2 est stopper de passer dans les moyens de perfusion 4 par le moyen d'obturation 11 avant de pénétrer dans les moyens de perfusion 4.

Ce procédé est particulièrement adapté dans le cas où la première solution contient un médicament et la deuxième solution est une solution de rinçage.

En effet, les médicaments tels que les antibiotiques, antimitotiques, anesthésiants ou agents de chimiothérapie nécessitent un dosage précis et doivent par conséquent être injectés dans leur totalité.

Lors de la perfusion de la première solution contenant le médicament, il subsiste des traces de médicament dans la première poche 2, la première tubulure 5 et les moyens de perfusion 4.

En faisant passer de la deuxième solution vers la première poche 2 puis en la perfusant, le dispositif de perfusion 1 est rincé dans sa totalité.

Ainsi, la combinaison des moyens de sélection 10 et du moyen d'obturation 11 permet, outre l'empêchement, sans manipulation supplémentaire, de toute administration d'air, un meilleur rinçage du dispositif de perfusion puisque la solution de rinçage est introduite dans toutes les tubulures ainsi que dans la première poche 2.

Selon une variante de l'invention, le procédé de perfusion comprend une étape préalable de commande des moyens de sélection 10 pour perfuser de la deuxième solution depuis la deuxième poche 3.

En particulier, cette étape autorise la purge préalable du dispositif de perfusion et de l'abord veineux du patient avec de la solution de rinçage avant administration de la solution médicamenteuse contenue dans la première poche 2.

## Revendications

1. Dispositif de perfusion (1) formant un système unitaire en circuit fermé, comprenant une première poche (2) contenant ou destinée à contenir une première solution, une deuxième poche (3) contenant ou destinée à contenir une deuxième solution, et des moyens de perfusion (4) en communication fluidique avec la première poche (2) par l'intermédiaire d'une première tubulure (5) branchée respectivement sur un orifice d'entrée/sortie (6) de ladite première poche et sur un moyen de connexion (7) sur lequel sont branchés les moyens de perfusion (4), la deuxième poche (3) étant en communication fluidique avec la première poche (2) par l'intermédiaire d'une deuxième tubulure (8) branchée respectivement sur un orifice de sortie (9) de ladite deuxième poche et sur le moyen de connexion (7), ledit dispositif étant **caractérisé en ce qu'**il comprend en combinaison :
- des moyens de sélection à commande externe (10) aptes à sélectionner le passage, dans les moyens de perfusion (4), de la solution contenue dans la première poche (2) ou le passage de la deuxième solution dans la première poche (2) ;
- un moyen d'obturation réversible (11) de l'orifice d'entrée/sortie (6) de la première poche (2), ledit moyen comprenant un organe flottant (12) qui est monté mobile, à l'intérieur de la première poche (2), en regard de l'orifice d'entrée/sortie (6) entre une position d'obturation dans l'ouverture dudit orifice et une position d'écoulement à distance dudit orifice.

2. Dispositif selon la revendication 1, **caractérisé en ce que** le moyen d'obturation (11) comprend un guide vertical ajouré (14) qui est prévu dans le prolongement de l'orifice d'entrée/sortie (6), dans lequel l'organe flottant (12) est logé mobile.

3. Dispositif selon la revendication 1 ou 2, **caractérisé en ce que** l'organe flottant (12) est formé d'une bille dont le diamètre est de l'ordre, tout en étant supérieur, à celui de l'ouverture de l'orifice d'entrée/sortie (6).

4. Dispositif selon l'une quelconque des revendications 1 à 3, **caractérisé en ce que** les moyens de perfusion (4) comprennent une troisième tubulure (17) dont une extrémité est connectée au moyen de connexion (7) et dont l'autre extrémité comprend un moyen de raccordement au circuit veineux du patient (18).

5. Dispositif selon la revendication 4, **caractérisé en ce que** les moyens de perfusion (4) comprennent en outre une chambre compte-gouttes (19) qui est disposée sur la troisième tubulure (17).

6. Dispositif selon l'une quelconque des revendications 1 à 5, **caractérisé en ce que** le moyen de connexion (7) et les moyens de sélection (10) sont intégrés sous la forme d'un robinet à voies multiples (21).

7. Dispositif selon l'une quelconque des revendications 1 à 5, **caractérisé en ce que** le moyen de connexion (7) est formé d'une jonction trois voies (22).

8. Dispositif selon la revendication 7, **caractérisé en ce que** les moyens de sélection (10) comprennent au moins un organe de pincement de type clamp (25, 26) ou roulette (27) qui est disposé sur une tubulure.

9. Dispositif selon la revendication 7 ou 8, **caractérisé en ce que** les moyens de sélection (10) comprennent un organe sécable de type ouvre-circuit (23, 24) qui est disposé dans une tubulure.

10. Dispositif selon l'une quelconque des revendications 1 à 9, **caractérisé en ce que** les moyens de sélection (10) sont en outre aptes à sélectionner le passage, dans les moyens de perfusion (4), de la deuxième solution lorsqu'elle est contenue dans la deuxième poche (3).

11. Dispositif selon l'une quelconque des revendications 1 à 10, dans lequel la première solution contient un médicament et la deuxième solution est une solution de rinçage.
